# EUROPEAN PATENT SPECIFICATION

(11) **EP 2 775 973 B1**
(45) Date of publication and mention of the grant of the patent: **09.05.2018**
(21) Application number: 11782067.0
(22) Date of filing: 10.11.2011
(51) Int. Cl.: A61F 9/008

(54) **LASER-ASSISTED EPITHELIAL REMOVAL**
LASERUNTERSTÜTZTE EPITHELENTFERNUNG
ÉLIMINATION D'ÉPITHÉLIUM ASSISTÉE PAR LASER

(43) Date of publication of application: 17.09.2014
(73) Proprietor: WaveLight GmbH, 91058 Erlangen (DE)
(72) Inventor: DONITZKY, Christof, 90542 Eckental/Eschenau (DE); KLENKE, Jörg, 90425 Nürnberg (DE)
(74) Representative: Katérle, Axel
(86) International application number: PCT/EP2011/005659
(87) International publication number: WO 2013/068025

(56) References cited:
- WO-A2-2004/026198
- WO-A2-2007/143111
- US-A1- 2009 247 999
- US-A1- 2010 210 996

## Description

### TECHNICAL FIELD

The present disclosure relates generally to corneal surgical devices, and more particularly to laser-assisted epithelial removal.

### BACKGROUND

Refractive surgery typically reshapes the cornea to correct refractive defects in the eye. In some types of refractive surgery, the epithelium of the cornea is detached from the Bowman's layer, and then the Bowman's layer together with the corneal stroma is shaped to apply the refractive correction.

There are several known techniques for removing the epithelium, but these techniques can yield poor results that adversely affect the shaping process and/or lengthen the recovery time. For example, in photorefractive keratectomy (PRK), a surgical instrument (such as a hockey knife) is used to remove the epithelium. The force used to detach the epithelium, however, can traumatize the cornea. In addition, the surgical instrument can damage, roughen, or tear the Bowman's layer. Moreover, the surgeon might create a larger surgical zone than is optically necessary. As another example, in Laser Assisted Sub-Epithelial Keratomileusis (LASEK), an alcohol solution is used to weaken epithelial cells so they can be manually removed. The alcohol solution, however, can dry out the Bowman's layer and change the ablation rate of the layer, which affects how the desired correction should be applied. The alcohol solution can also delay healing. As yet another example, in Epi-Lasik, a separator is used to detach the epithelium from the Bowman's layer. The separator, however, can damage the Bowman's layer. As a last example, an excimer laser can be used to remove the epithelium and shape the cornea. The excimer laser, however, does not perform optimally in certain situations. As yet another example, documents WO2004/026198, WO2007/143111, US2009/0247999 and US2010/0210996 disclose femtosecond laser systems for removing the epithelial layer of the cornea.

### BRIEF SUMMARY

In certain embodiments, a device configured to perform epithelial removal comprises a laser device and a control computer. The laser device can separate the epithelium from the Bowman's layer using pulsed laser radiation having ultrashort pulses (such as pico-, femto-, or attosecond pulses). The laser device includes controllable components that control a focus of the pulsed laser radiation. The control computer instructs the controllable components to focus the pulsed laser radiation at the Basal cell layer of the epithelium to photodisrupt at least a portion of the basal cell layer. A method for performing epithelial removal, the method not being part of this invention, includes focusing pulsed laser radiation at an epithelial cell layer of the epithelium of an eye. The pulsed laser radiation has ultrashort pulses. At least a portion of the epithelial cell layer is photodisrupted, and the epithelium is separated from the Bowman's layer of the eye.

In certain embodiments, a tangible computer-readable medium stores computer code for performing epithelial removal by focusing pulsed laser radiation at a basal cell layer of the epithelium of an eye. The pulsed laser radiation has ultrashort pulses. At least a portion of the basal cell layer is photodisrupted, and the epithelium is separated from the Bowman's layer of the eye. The invention is defined in the claims.

### BRIEF DESCRIPTION OF THE DRAWINGS

Exemplary embodiments of the present disclosure will now be described by way of example in greater detail with reference to the attached figures, in which:
FIGURES 1A and 1B illustrate examples of devices configured to perform epithelial removal according to certain embodiments;
FIGURES 2A through 2C illustrate an example of a cell layer of the epithelium of a cornea that may be photodisrupted according to certain embodiments;
FIGURES 3 and 4 illustrate examples of epithelial elements that may be created from a cornea according to certain embodiments;
FIGURE 5 illustrates a cross section of an example of a bed incision and examples of lateral incisions; and
FIGURES 6A and 6B illustrate examples of forming a bed incision and forming a lateral incision.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit or restrict the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments. In addition, certain drawings may be in schematic form.

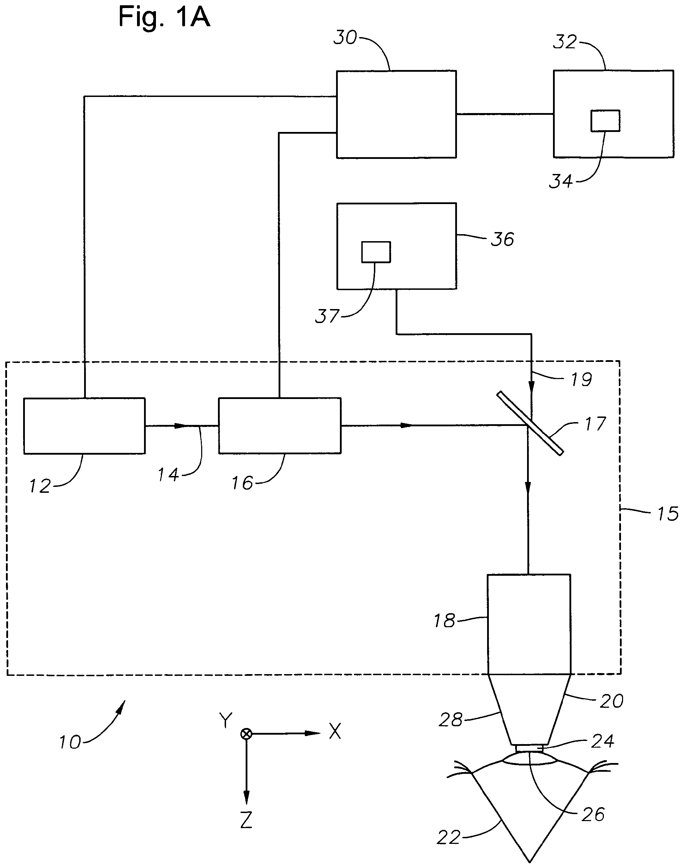

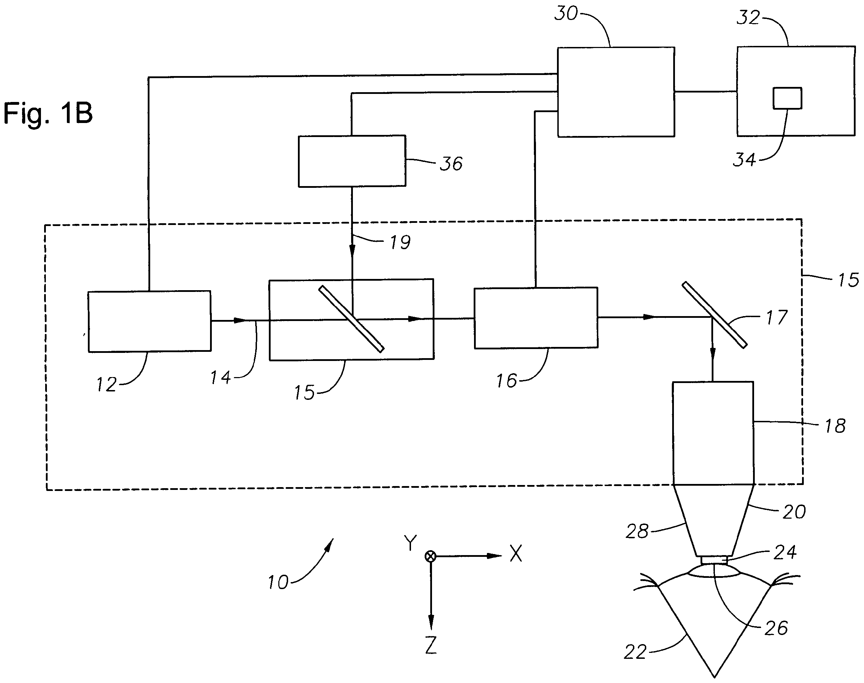

FIGURES 1A and 1B illustrate examples of devices 10 configured to perform epithelial removal according to certain embodiments. In the embodiments, the device 10 includes a laser device and a control computer. The laser device can separate the epithelium of the cornea from the Bowman's layer using pulsed laser radiation with ultrashort pulses (such as pico-, femto-, or attosecond pulses). The laser device may include controllable components that focus the pulsed laser radiation. The control computer instructs the controllable components to focus the pulsed laser radiation at a cell layer of the epithelium to destroy at least a portion of the layer to separate the epithelium from the Bowman's layer. An excimer laser can then be used to reshape the Bowman's layer and upper stroma of the cornea to apply a refractive correction. In certain embodiments, the separated epithelium forms an epithelial element (such as an epithelial flap or epithelial cap), which may or may not be replaced after the refractive correction. In other embodiments, the epithelium is removed completely from the cornea, e.g., with a suitable surgical instrument such as a stype, pad, or sponge.

In the illustrated example of FIGURE 1A, the device 10 performs surgery on an eye 22. The device 10 includes a laser device 15, a patient adapter 20, a control computer 30, a memory 32, and an optical coherence tomography (OCT) system 36 (with an integrated scanner 37) coupled as shown. The OCT system 36 may or may not be coupled to the control computer 30. The laser device 15 may include a laser source 12, a scanner 16, one or more optical elements 17, and/or a focusing objective 18 coupled as shown. The patient adapter 20 may include a contact element 24 (which has an abutment face 26 disposed outwardly from a sample) and a sleeve 28 coupled as shown. The memory 32 stores a control program 34. The sample may be an eye 22 or a probe.

The laser source 12 generates a laser beam 14 with ultrashort pulses. In this document, an "ultrashort" pulse of light refers to a light pulse that has a duration that is less than a nanosecond, such as on the order of a picosecond, femtosecond, or attosecond. The focal point of the laser beam 14 may create a laser-induced optical breakdown (LIOB) in tissues such as the cornea. The laser beam 14 may be precisely focused to allow for precise incisions in the epithelial cell layers, which may reduce or avoid unnecessary destruction of other tissue.

Examples of laser source 12 include femtosecond, picosecond, and attosecond lasers. The laser beam 14 may have any suitable vacuum wavelength, such as a wavelength in the range of 300 to 1500 nanometers (nm), for example, a wavelength in the range of 300 to 650, 650 to 1050, 1050 to 1250, or 1100 to 1500 nm. The laser beam 14 may also have a relatively small focus volume, e.g., 5 micrometers (µm) or less in diameter. In certain embodiments, the laser source 12 and/or delivery channel may be in a vacuum or near vacuum.

The scanner 16, optical elements 17, and focusing objective 18 are in the beam path. The scanner 16 transversely and longitudinally controls the focal point of the laser beam 14. "Transverse" refers to a direction at right angles to the direction of propagation of the laser beam 14, and "longitudinal" refers to the direction of beam propagation. The transverse plane may be designated as the x-y plane, and the longitudinal direction may be designated as the z-direction. In certain embodiments, the abutment face 26 of the patient interface 20 is on an x-y plane.

The scanner 16 may transversely direct the laser beam 14 in any suitable manner. For example, the scanner 16 may include a pair of galvanometrically actuated scanner mirrors that can be tilted about mutually perpendicular axes. As another example, the scanner 16 may include an electro-optical crystal that can electro-optically steer the laser beam 14. The scanner 16 may longitudinally direct the laser beam 14 in any suitable manner. For example, the scanner 16 may include a longitudinally adjustable lens, a lens of variable refractive power, or a deformable mirror that can control the z-position of the beam focus. The focus control components of the scanner 16 may be arranged in any suitable manner along the beam path, e.g., in the same or different modular units.

One (or more) optical elements 17 direct the laser beam 14 towards the focusing objective 18. An optical element 17 may be any suitable optical element that can reflect and/or refract/diffract the laser beam 14. For example, an optical element 17 may be an immovable deviating mirror. The focusing objective 18 focuses the laser beam 14 onto the patient adapter 20, and may be separably coupled to the patient adapter 20. The focusing objective 18 may be any suitable optical element, such as an f-theta objective.

Patient adapter 20 interfaces with the cornea of the eye 22. In the example, the patient adapter 20 has a sleeve 28 coupled to a contact element 24. The sleeve 28 couples to the focusing objective 18. The contact element 24 is transparent to the laser radiation and has an abutment face 26 that interfaces with the cornea and may level a portion of the cornea. In certain embodiments, the abutment face 26 is planar and forms a planar area on the cornea. The abutment face 26 may be on an x-y plane, so the planar area is also on an x-y plane. In other embodiments, the cornea need not have planar area.

The control computer 30 controls controllable components, e.g., the laser source 12 and scanner 16, in accordance with the control program 34. The control program 34 contains computer code that instructs the controllable components to focus the pulsed laser radiation at an epithelial cell layer of the epithelium to photodisrupt at least a portion of the layer. The photodisruption forms a separation between the epithelial cell layer and the rest of the cornea.

In certain examples of operation, the scanner 16 may direct the laser beam 14 to form incisions of any suitable geometry. Examples of types of incisions include bed incisions and lateral incisions. A bed incision (e.g., an "epithelial flap bed incision") is two-dimensional incision that is typically on an x-y plane. The scanner 16 may form a bed incision by focusing the laser beam 14 at a constant z-value under the abutment face 26 and moving the focus in a pattern in an x-y plane. A lateral incision is an incision that extends from under the corneal surface (such as from a bed incision) to the surface. The scanner 16 may form a lateral incision by changing the z-value of the focus of the laser beam 14 and optionally changing the x and/or y values.

In certain embodiments, the control computer 30 determines the depth of the epithelial cell layer and instructs the controllable components to focus the laser beam 14 to form a bed incision at that depth. The depth may be determined in any suitable manner. For example, a user (such as a surgeon) may input the depth, which is received by the control computer 30.

In certain embodiments, the optical coherence tomography (OCT) system 36 measures the depth of the epithelial cell layer and sends the depth to the control computer 30. The scanner 37 of the OCT system 36 may direct a measurement beam 19 towards optical elements 17, which direct the beam 19 towards the eye 22 to measure the eye 22. The OCT system 36 uses low coherence interferometry to determine the location of parts of the eye 22 (e.g., the Epithelium, Bowman's layer, Stroma, Decement's membrane, and/or Endothelium), and may have a resolution of less than one (1) micrometer (µm). The laser beam 14 and measurement beam 19 may be used at the same time or may be used at different times.

In the illustrated example of FIGURE 1B, the device 10 includes a beam splitter 15, towards which the OCT system 36 directs the measurement beam 19. The OCT system 35 may or may not be coupled to the control computer 30.

In certain embodiments, the beam splitter 15 switches between the laser beam 14 and measurement beam 19 to allow both the laser beam 14 and measurement beam 19 to use the scanner 16. The beam splitter 15 may have any suitable features to switch from one beam to another beam, e.g., the beam splitter 15 may include at least one movable mirror or a dielectric coating and/or may be coupled to a movable device such as a carriage or a controllable arm.

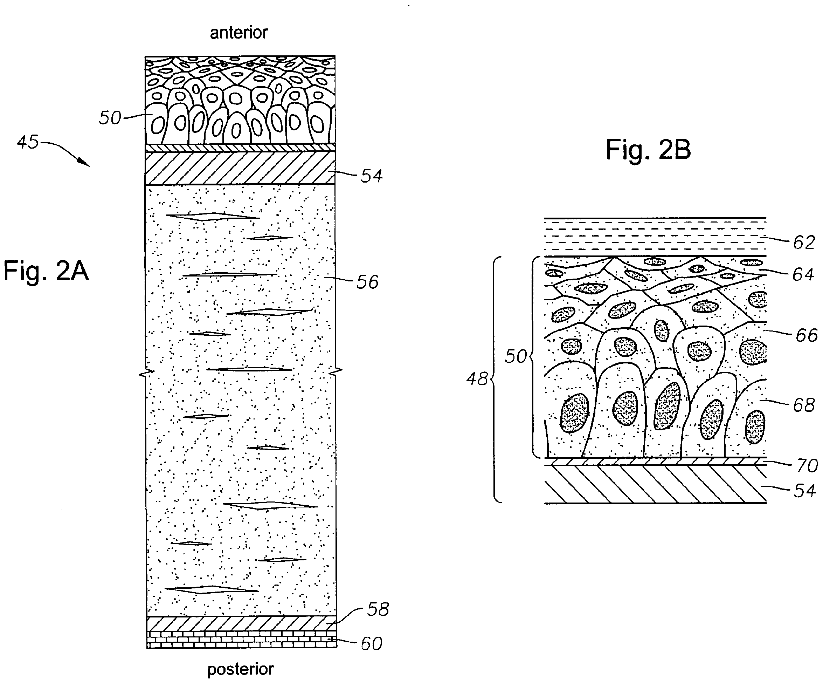

FIGURES 2A through 2C illustrate an example of an epithelial cell layer of the epithelium of a cornea that may be photodisrupted according to certain embodiments of the invention. FIGURE 2A illustrates the layers 45 of a cornea. Layers 45 include the epithelium (or Epithelium) 50, Bowman's layer 54, stroma (or Stroma) 56, Descemet's membrane 58, and endothelium (or Endothelium) 60. FIGURE 2B illustrates a precorneal tear film 62 and a subset 48 of the corneal layers 45. The subset 48 includes the epithelium 50 and Bowman's layer 54. The epithelium 50 includes the following cell layers: the squamous cells 64, wing cells 66, basal cells 68, and basement membrane 70.

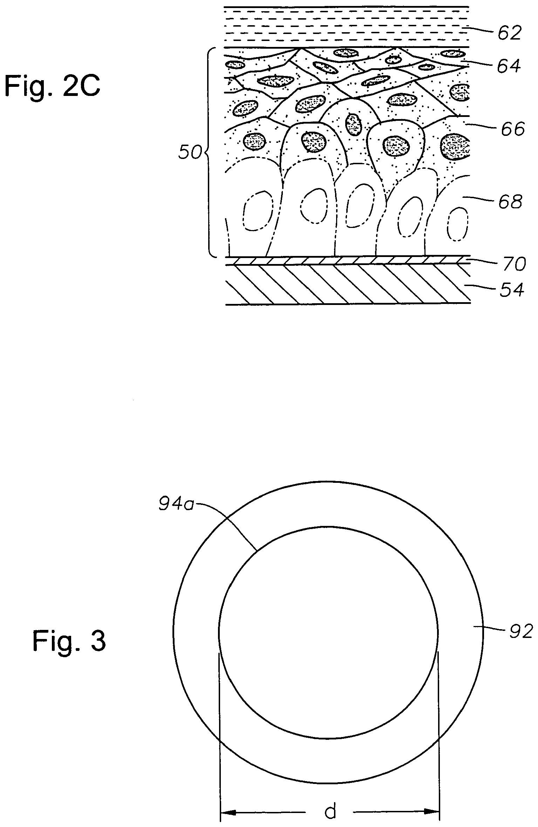

Any suitable portion of the epithelium 50 may be photodisrupted. One or more of any of the epithelial cell layers may be selected for photodisruption. For example, basal cells 68 or basal cells 68 and wing cells 66 may be photodisrupted. (In the example of FIGURE 2C, the basal cell layer is destroyed.) In addition, a portion of a cell layer may be photodisrupted in the z-direction, but part of the cell layer may remain on the cornea. For example, some posterior wing cells 66 may be destroyed, but some anterior wing cells 66 may remain. Moreover, a particular area (or "target zone") in the x-y plane may be selected for photodisruption. For example, a target zone that forms the bed of an epithelial element may be photodisrupted. Photodisruption of a layer the epithelium 50 may be regarded as creating a separation between the epithelium 50 and the rest of the cornea and thus separating the epithelium 50 from the cornea.

The device 10 may photodisrupt an epithelial cell layer in any suitable manner. In certain embodiments, the control computer 30 may instruct the laser device to focus the laser beam 14 at a constant z-value under the abutment face 26 and move in a pattern in the x-y plane that substantially covers the target zone. Any suitable pattern may be used. For example, according to a zigzag pattern, the scan path has a constant y-value and moves in the +x direction. When the scan path reaches a point of the border of the target zone, the path moves to a next y value that is a predetermined distance from the previous y-value and then moves in the -x direction until it reaches another point of the border. The scan path continues until the entire target zone is scanned. As another example, according to a spiral pattern, the scan path starts at or near the center of the target zone and moves in a spiral pattern until the path reaches the border of the target zone, or vice-versa.

As the laser beam 14 travels along the scan path, the laser beam pulses create microdisruptions. In certain situations, a scan path pattern may yield a nonuniform distribution of microdisruptions over the target zone. In these cases, the laser beam 14 may be modified to make the distribution more uniform. For example, certain pulses may be blocked or the pulse energy may be decreased to reduce number of or the effect of the pulses in a particular region.

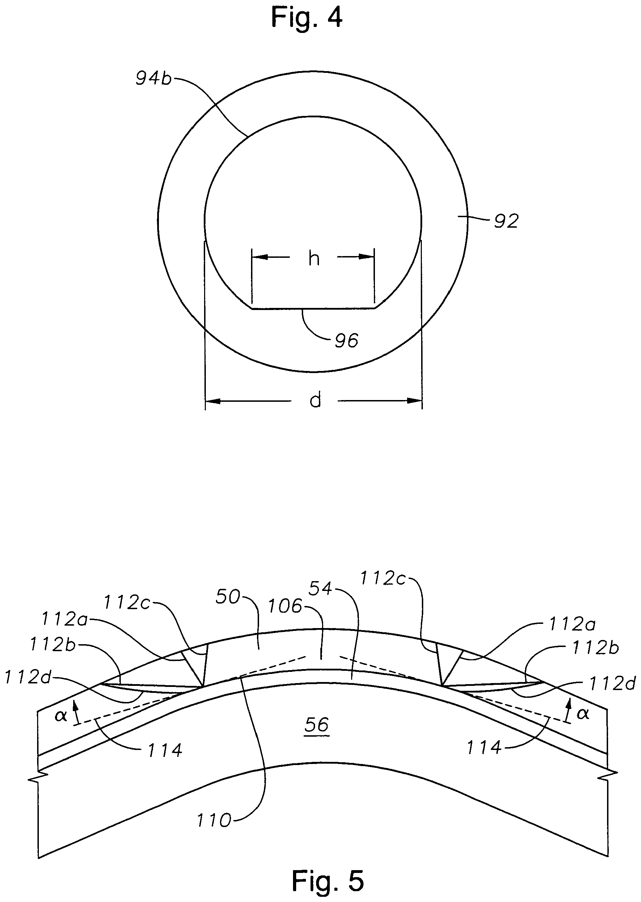

FIGURES 3 and 4 illustrate examples of epithelial elements 94 (94a-b) that may be created from a cornea 92 according to certain embodiments. The epithelial elements 94 include an epithelial cap 94a that can be removed entirely from the cornea 92 and an epithelial flap 94b that has a hinge 96 that connects the flap 94b to the rest of the epithelium or the Basement membrane 70.

The epithelial element 94 may have any suitable size and shape. For example, the epithelial element 94 may have any suitable diameter d and thickness. As another example, the epithelial element 94 may have any suitable shape, e.g., a circular, elliptical, free form, or irregular shape. As an example relating to the epithelial flap 94, the hinge 96 may have any suitable length h.

The device 10 may create the epithelial element 94 in any suitable manner. In certain embodiments, the control computer 30 may instruct the laser device to form a bed incision and a lateral incision. The bed incision severs the epithelial element 94 from the Bowman's layer 54. The depth of the bed incision corresponds to the thickness of the flap 94. The lateral incision extends from the bed incision to the surface of the cornea and follows the outline of the epithelial flap 94. To create an epithelial cap 94a, the lateral incision forms a closed loop around the cap 94a. To create an epithelial flap 94b, the lateral incision excludes the hinge 96.

After creation of the epithelial element 94, the element 94 can be removed to allow for the correction process. For example, a cap 94a can be completely removed, or a flap 94b can be lifted and folded back. An excimer laser can provide laser radiation through the Bowman's layer 54 to reshape the Bowman's layer 54 and upper stroma 50 in order to apply a refractive correction. If a cap 94a was used, the epithelium layers will grow back during the healing process. If a flap 94b was used, the flap 94b can be replaced after the refractive correction, and re-growth may occur.

FIGURE 5 illustrates a cross section of an example of a bed incision 110 and examples of lateral incisions 112 (112a-d). The bed incision 110 is formed under the epithelium to be removed 106. A lateral incision 112 may have any suitable angle a from a tangent 114 that is tangent to the Bowman's layer 54, where the angle a goes in a direction towards the epithelium to be removed 106. For example, the angle a may have a value in the range of 0 to 135°, such as 0 to 30, 30 to 60, 60 to 90, 90 to 120, or 120 to 135°. In the illustration, the lateral incision 112a has an angle a of approximately 85°, the lateral incision 112b has an angle a of approximately 30°, and the lateral incision 112c has an angle a of approximately 135°.

In addition, the cross section of a lateral incision 112 may have any suitable shape. In the example, the cross sections of lateral incisions 112a-c are lines, and the cross section of the lateral incision 112d is a curve, which is any set of continuous points that does not cross itself. The cross section of a lateral incision 112, however, may have any suitable shape, and may, e.g., be discontinuous at one or more points or may cross itself at one or more points.

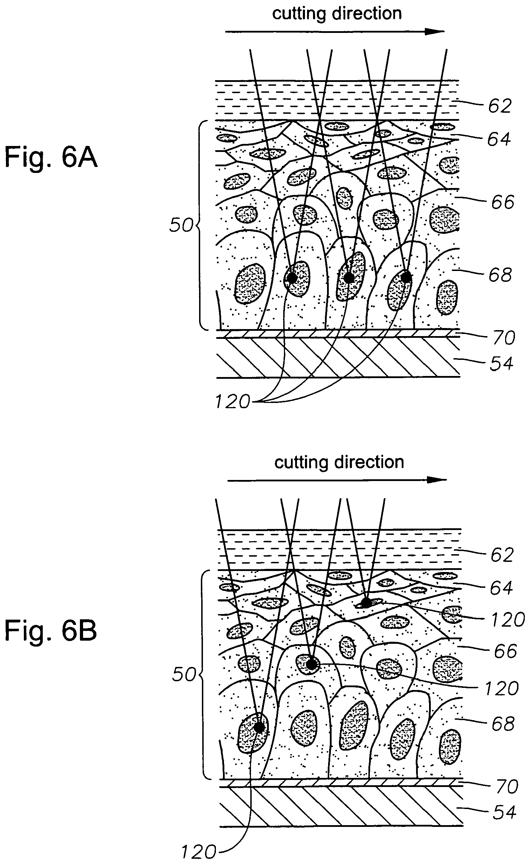

FIGURE 6A illustrates an example of forming a bed incision, and FIGURE 6B illustrates an example of forming a lateral incision. As discussed above, an incision is formed by directing the focal point 120 of the laser beam to the location of the incision in order to cut the incision. The laser beam can be precisely focused, so the incisions can be precisely formed.

A component (such as the control computer 30) of the systems and apparatuses disclosed herein may include an interface, logic, memory, and/or other suitable element, any of which may include hardware and/or software. An interface can receive input, send output, process the input and/or output, and/or perform other suitable operations. Logic can perform the operations of a component, for example, execute instructions to generate output from input. Logic may be encoded in memory and may perform operations when executed by a computer. Logic may be a processor, such as one or more computers, one or more microprocessors, one or more applications, and/or other logic. A memory can store information and may comprise one or more tangible, computer-readable, and/or computer-executable storage medium. Examples of memory include computer memory (for example, Random Access Memory (RAM) or Read Only Memory (ROM)), mass storage media (for example, a hard disk), removable storage media (for example, a Compact Disk (CD) or a Digital Video or Versatile Disk (DVD)), database and/or network storage (for example, a server), and/or other computer-readable media.

In particular embodiments, operations of the embodiments may be performed by one or more computer readable media encoded with a computer program, software, computer executable instructions, and/or instructions capable of being executed by a computer. In particular embodiments, the operations may be performed by one or more computer readable media storing, embodied with, and/or encoded with a computer program and/or having a stored and/or an encoded computer program.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, and the operations of the systems and apparatuses may be performed by more, fewer, or other components. As another example, modifications may be made to the methods disclosed herein. The methods may include more, fewer, or other steps, and the steps may be performed in any suitable order.

Other modifications are possible without departing from the scope of the invention. For example, the description illustrates embodiments in particular practical applications, yet other applications will be apparent to those skilled in the art. In addition, future developments will occur in the arts discussed herein, and the disclosed systems, apparatuses, and methods will be utilized with such future developments.

The scope of the invention should not be determined with reference to the description. In accordance with patent statutes, the description explains and illustrates the principles and modes of operation of the invention using exemplary embodiments. The description enables others skilled in the art to utilize the systems, apparatuses, and methods in various embodiments and with various modifications, but should not be used to determine the scope of the invention.

The scope of the invention should be determined with reference to the claims and the full scope of equivalents to which the claims are entitled. All claims terms should be given their broadest reasonable constructions and their ordinary meanings as understood by those skilled in the art, unless an explicit indication to the contrary is made herein. For example, use of the singular articles such as "a," "the," etc. should be read to recite one or more of the indicated elements, unless a claim recites an explicit limitation to the contrary. As another example, "each" refers to each member of a set or each member of a subset of a set, where a set may include zero, one, or more than one element. In sum, the invention is capable of modification, and the scope of the invention should be determined, not with reference to the description, but with reference to the claims and their full scope of equivalents.

## Claims

1. A device configured to perform epithelial removal, the device comprising:
a laser device configured to separate an epithelium from a Bowman's layer of an eye using pulsed laser radiation having a plurality of ultrashort pulses, the laser device comprising one or more controllable components configured to control a focus of the pulsed laser radiation; **characterised by** a control computer configured to instruct the one or more controllable components to:
create a bed incision that severs an epithelial flap or cap from the Bowman's layer by focusing the pulsed laser radiation at a basal cell layer of the epithelium to photodisrupt at least a portion of the basal cell layer.

2. The device of Claim 1, the control computer configured to instruct the one or more controllable components to focus the pulsed laser radiation at the basal cell layer by:
receiving an input designating a depth of the basal cell layer; and
instructing the one or more controllable components to focus the pulsed laser radiation at the depth.

3. The device of Claim 1 or 2, further comprising:
an optical coherence tomography (OCT) system configured to measure a depth of the basal cell layer and send the depth to the control computer.

4. The device of any one preceding Claim, an ultrashort pulse having a pulse that is less than one nanosecond.

5. One or more tangible computer-readable media storing computer code that when executed by a computer is configured to:
focus pulsed laser radiation from a laser device at a basal cell layer of an epithelium of an eye, the pulsed laser radiation having a plurality of ultrashort pulses; **characterised in that** the one or more tangible computer-readable media storing computer code that when executed by a computer is further configured to photodisrupt at least a portion of the basal cell layer to create a bed incision that severs an epithelial flap or cap from a Bowman's layer of the eye; and
separate the epithelium from the Bowman's layer.

6. The tangible computer-readable media of Claim 5, the focusing the pulsed laser radiation further comprising:
receiving an input designating a depth of the basal cell layer; and
focusing the pulsed laser radiation at the depth.

7. The tangible computer-readable media of Claim 5, the focusing the pulsed laser radiation further comprising:
measuring a depth of the basal cell layer using an optical coherence tomography (OCT) system; and
focusing the pulsed laser radiation at the depth.

8. The tangible computer-readable media of Claim 5, an ultrashort pulse having a pulse that is less than one nanosecond.

## Patentansprüche

1. Vorrichtung, gestaltet zum Durchführen einer Epithelentfernung, wobei die Vorrichtung umfasst:
eine Laservorrichtung, gestaltet zum Abtrennen eines Epithels von einer Bowman-Schicht eines Auges unter Verwendung von gepulster Laserstrahlung mit einer Vielzahl von ultrakurzen Pulsen, wobei die Laservorrichtung eine oder mehrere steuerbare Komponenten umfasst, die dafür gestaltet sind, einen Fokus der gepulsten Laserstrahlung zu steuern; **gekennzeichnet durch**
einen Steuercomputer, der dafür gestaltet ist, die eine oder mehreren steuerbaren Komponenten anzuweisen:
einen Bettschnitt zu erzeugen, der einen/eine Epithellappen oder -kappe von der Bowman-Schicht abtrennt, indem die gepulste Laserstrahlung auf eine Basalzellenschicht des Epithels fokussiert wird, um wenigstens einen Teil der Basalzellenschicht durch Licht zu zertrennen.

2. Vorrichtung gemäß Anspruch 1, wobei der Steuercomputer dafür gestaltet ist, die eine oder mehreren steuerbaren Komponenten anzuweisen, die gepulste Laserstrahlung auf die Basalzellenschicht zu fokussieren durch:
Empfangen eines Eingangswerts, der eine Tiefe der Basalzellenschicht angibt; und
Anweisen der einen oder mehreren steuerbaren Komponenten, die gepulste Laserstrahlung auf die Tiefe zu fokussieren.

3. Vorrichtung gemäß Anspruch 1 oder 2, ferner umfassend:
ein optische-Kohärenztomographie(OCT)-System, das dafür gestaltet ist, eine Tiefe der Basalzellenschicht zu messen und die Tiefe an den Steuercomputer zu übermitteln.

4. Vorrichtung gemäß einem der vorstehenden Ansprüche, wobei ein ultrakurzer Puls einen Puls aufweist, der kürzer als eine Nanosekunde ist.

5. Ein oder mehrere materielle computerlesbare Medien, die Computercode speichern, der dafür gestaltet ist, wenn von einem Computer ausgeführt:
gepulste Laserstrahlung von einer Laservorrichtung auf eine Basalzellenschicht eines Epithels eines Auges zu fokussieren, wobei die gepulste Laserstrahlung eine Vielzahl von ultrakurzen Pulsen aufweist;
**dadurch gekennzeichnet, dass** das eine oder die mehreren materiellen computerlesbaren Medien Computercode speichern, der ferner dafür gestaltet ist, wenn von einem Computer ausgeführt,
durch Licht wenigstens einen Teil der Basalzellenschicht zu zertrennen, um einen Bettschnitt zu erzeugen, der einen/eine Epithellappen oder -kappe von einer Bowman-Schicht des Auges abtrennt; und
das Epithel von der Bowman-Schicht zu trennen.

6. Materielles computerlesbares Medium gemäß Anspruch 5, wobei das Fokussieren der gepulsten Laserstrahlung ferner umfasst:
Empfangen eines Eingangswerts, der eine Tiefe der Basalzellenschicht angibt; und
Fokussieren der gepulsten Laserstrahlung auf die Tiefe.

7. Materielles computerlesbares Medium gemäß Anspruch 5, wobei das Fokussieren der gepulsten Laserstrahlung ferner umfasst:
Messen einer Tiefe der Basalzellenschicht unter Verwendung eines optische-Kohärenztomographie(OCT)-Systems; und
Fokussieren der gepulsten Laserstrahlung auf die Tiefe.

8. Materielles computerlesbares Medium gemäß Anspruch 5, wobei ein ultrakurzer Puls einen Puls aufweist, der kürzer als eine Nanosekunde ist.

## Revendications

1. Dispositif configuré pour effectuer une élimination d'épithélium, le dispositif comprenant :
un dispositif laser configuré pour séparer un épithélium d'une couche de Bowman d'un oeil à l'aide d'un rayonnement laser pulsé ayant une pluralité d'impulsions ultracourtes, le dispositif laser comprenant un ou plusieurs composants commandables, configurés pour commander une focalisation du rayonnement laser pulsé ; **caractérisé en ce qu'**il comprend
un ordinateur de commande configuré pour donner l'instruction à un ou plusieurs composants commandables de :
créer une incision de couche qui coupe un volet ou un capuchon épithélial de la couche de Bowman en focalisant le rayonnement laser pulsé au niveau d'une couche cellulaire de base de l'épithélium pour photocautériser au moins une partie de la couche cellulaire de base.

2. Dispositif selon la revendication 1, l'ordinateur de commande étant configuré pour donner l'instruction à un ou plusieurs composants commandables de focaliser le rayonnement laser pulsé au niveau de la couche cellulaire de base par :
la réception d'une entrée désignant une profondeur de la couche cellulaire de base ; et
l'instruction du ou des composants commandables de focaliser le rayonnement laser pulsé à la profondeur.

3. Dispositif selon la revendication 1 ou 2, comprenant en outre :
un système de tomographie par cohérence optique (OCT) configuré pour mesurer une profondeur de la couche cellulaire de base et envoyer la profondeur à l'ordinateur de commande.

4. Dispositif selon l'une quelconque des revendications précédentes, une impulsion ultracourte ayant une impulsion qui est inférieure à une nanoseconde.

5. Un ou plusieurs supports perceptibles lisibles par ordinateur stockant un code informatique qui, lorsqu'il est exécuté par un ordinateur, est configuré pour :
focaliser un rayonnement laser pulsé à partir d'un dispositif laser au niveau d'une couche cellulaire de base d'un épithélium d'un oeil, le rayonnement laser pulsé ayant une pluralité d'impulsions ultracourtes ;
**caractérisés en ce que** le ou les supports perceptibles lisibles par ordinateur stockant un code informatique qui, lorsqu'il est exécuté par un ordinateur, est en outre configuré pour photocautériser au moins une partie de la couche cellulaire de base pour créer une incision de couche qui coupe un volet ou un capuchon épithélial à partir d'une couche de Bowman de l'oeil ; et
séparer l'épithélium de la couche de Bowman.

6. Support perceptible lisible par ordinateur selon la revendication 5, la focalisation du rayonnement laser pulsé comprenant en outre :
la réception d'une entrée désignant une profondeur de la couche cellulaire de base ; et
la focalisation du rayonnement laser pulsé à la profondeur.

7. Support perceptible lisible par ordinateur selon la revendication 5, la focalisation du rayonnement laser pulsé comprenant en outre :
la mesure d'une profondeur de la couche cellulaire de base à l'aide d'un système de tomographie par cohérence optique (OCT) ; et
la focalisation du rayonnement laser pulsé à la profondeur.

8. Support perceptible lisible par ordinateur selon la revendication 5, une impulsion ultracourte ayant une impulsion qui est inférieure à une nanoseconde.
